(19)

Europäisches Patentamt
European Patent Office
Office européen des brevets

(11) **EP 3 586 919 A1**

(12) **EUROPEAN PATENT APPLICATION**

(43) Date of publication:
**01.01.2020 Bulletin 2020/01**

(51) Int Cl.:
**A61N 5/10** *(2006.01)*     **A61N 5/06** *(2006.01)*

(21) Application number: **18180559.9**

(22) Date of filing: **28.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **VAN EE, Raymond**
**5656 AE Eindhoven (NL)**

• **TEN KATE, Warner Rudolph Theophile**
**5656 AE Eindhoven (NL)**
• **VAN DE WOUW, Doortje**
**5656 AE Eindhoven (NL)**
• **DONGRE, Chaitanya**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **METHOD, COMPUTER PROGRAM PRODUCT AND AN APPARATUS FOR DETERMINING A TREATMENT TIME WINDOW FOR ADMINISTERING A CANCER TREATMENT TO A PATIENT**

(57)     According to an aspect, there is provided an apparatus for determining a treatment time window for administering a cancer treatment to a patient for whom a maximum rate of healthy tissue cell division occurs at the same time or at approximately the same time as a maximum rate of cancerous tissue cell division, wherein the timing of the maximum rate of healthy tissue cell division is related to a circadian rhythm of the patient. The apparatus comprises a processing unit configured to determine one or more treatment time windows for administering the cancer treatment to the patient after the initiation of a desired phase shift in the circadian rhythm based on a shift factor indicating a rate at which the timing of the maximum rate of healthy tissue cell division shifts within a 24-hour cycle in response to the initiation of the desired phase shift, and a delay factor indicating a delay from a start of a phase shift in the circadian rhythm of the patient to the start of a corresponding phase shift in the timing of the maximum rate of cancerous tissue cell division; and provide an output to a user of the apparatus indicating the determined one or more treatment time windows.

Fig. 1

EP 3 586 919 A1

**Description**

FIELD OF THE INVENTION

This disclosure relates to a method, computer program product and an apparatus for determining a treatment time window for administering a cancer treatment to a patient.

BACKGROUND OF THE INVENTION

The rate of cell division in healthy human body tissue typically follows a 24-hour rhythm, referred to as the circadian rhythm. That is, the rate of cell division for healthy cells varies throughout the day. The circadian rhythm of a person is typically dependent on the solar day-night cycle. The circadian rhythm is also referred to as the biological clock (in the suprachiasmatic nucleus), and can be influenced by behavioural rhythms such as, for example, an eating schedule or a sports schedule.

Cancerous cells (also referred to as tumour cells) can have their own autonomous rhythm of cell division that is independent of the solar circadian light-dark rhythm. However, in some cases the rhythm of cell division for cancerous cells can be dependent on the solar circadian light-dark rhythm as the cell division rhythm for tumour cells can be influenced by the circadian rhythm of cell division for the healthy cells.

It is optimal to attack tumour cells with a medical intervention (like radiation or chemo therapy) during tumour cell division, since the tumour cells are more vulnerable and/or the cell division process itself can be targeted. However, such interventions may also affect healthy cells that are dividing and/or the healthy cell division process, and so in general it is better to attack tumour cells with a medical intervention when healthy cells are not themselves dividing. However this may not be possible where cell division of tumour cells follows an autonomous rhythm that is aligned with healthy cell division (i.e. where a high or maximum rate of cell division of tumour cells coincides with a high or maximum rate of cell division of healthy cells).

Therefore, for a patient where healthy tissue cell division occurs at the same time or at approximately the same time as cancerous tissue cell division (or where the time gap between the healthy tissue cell division and the cancerous tissue cell division is not sufficiently large to enable a cancer treatment that is to be administered for a particular amount of time without affecting the healthy tissue), there is a need for ways to determine a treatment time window for administering a cancer treatment to a patient that provides a balance between effective treatment of the cancerous tissue and minimising the damage to healthy tissue. Moreover, there is need for ways to create such a treatment time window for the patient to improve the treatment of the cancerous tissue and minimise damage to healthy tissue.

SUMMARY OF THE INVENTION

The techniques described herein are based on the insight that, for cancerous tissue where the timing of cancerous tissue cell division is dependent on, and initially aligned with, overlapping with, or generally too close in time to, the timing of healthy tissue cell division, when a phase shift in the circadian rhythm of the patient (and thus the timing of the healthy tissue cell division) is induced, the corresponding phase shift in the timing of the cancerous tissue cell division lags behind the corresponding phase shift in the timing of the healthy tissue cell division. This lag effectively creates a treatment window (or a larger treatment window) for the patient where the cancerous tissue cell division is not (or even less) aligned with the healthy tissue cell division, and a cancer treatment can be administered during this window with better effectiveness against the cancerous tissue (since the cancerous cells may be dividing), while minimising the damage caused to healthy tissue (since the healthy cells are not (or are less likely to be) dividing).

Thus, the techniques described herein provide a method, computer program product and apparatus that enable a suitable treatment time window to be derived for a desired phase shift that could be induced in the circadian rhythm of a patient, and, in some embodiments, a method, computer program product and apparatus is provided that can also cause or trigger such a phase shift as part of a treatment plan for the patient.

According to a first specific aspect, there is provided an apparatus for determining a treatment time window for administering a cancer treatment to a patient for whom a timing of a maximum rate of healthy tissue cell division is to be shifted with respect to a timing of a maximum rate of cancerous tissue cell division, wherein the timing of the maximum rate of healthy tissue cell division is based on a circadian rhythm of the patient. The apparatus comprises a processing unit configured to determine one or more treatment time windows for administering the cancer treatment to the patient after the initiation of a desired phase shift in the circadian rhythm based on a shift factor and a delay factor; and provide an output indicating the determined one or more treatment time windows. The shift factor indicates a rate at which the timing of the maximum rate of healthy tissue cell division shifts within a 24-hour cycle in response to the initiation of the desired phase shift. The delay factor indicates a delay from a start of a phase shift in the circadian rhythm of the patient to the start of a corresponding phase shift in the timing of the maximum rate of cancerous tissue cell division. Thus, the apparatus according to the first aspect provides that the timing of one or more treatment time windows in which a cancer treatment can be administered to attack the cancerous cells when they are dividing while minimising the risk of damaging healthy cells when they divide. This can enable the reduction of side effects to the patient (for example caused by damage to healthy tissue by the cancer treatment) and/or an increase in the effectiveness of the cancer treatment (for example if the dosage can be increased in the treatment time window due to the lower risk of unwanted side effects).

In some embodiments, the processing unit is further configured to initiate a phase shift in the circadian rhythm according to the desired phase shift. These embodiments have the advantage that, in addition to identifying when one or more treatment time windows might occur following the initiation of a phase shift, the apparatus can cause the phase shift to occur and create the treatment time windows.

In some embodiments, the desired phase shift is to be caused by the application of a light pattern in the environment of the patient. In these embodiments, the light pattern can be a day-night light pattern. In these embodiments, the light pattern can be a day-night light pattern associated with a different time zone to a time zone at a current location of the patient. In any of these embodiments, the processing unit can be further configured to control one or more light sources in the environment of the patient to apply the light pattern.

In alternative embodiments, the desired phase shift can be caused by the administration of a medication or substance to the patient. In these embodiments, the processing unit can be configured to control a medication dispenser to dispense the medication or substance to the patient to cause the desired phase shift.

In some embodiments, the processing unit is configured to determine the desired phase shift based on the shift factor and a desired number of days over which the cancer treatment is to be administered to the patient. This has the advantage that the phase shift to be applied to the patient can be customised to the length of the cancer treatment. In these embodiments, the processing unit can be configured to determine the desired phase shift as a phase shift that is higher than the product of the shift factor and the desired number of days over which the cancer treatment is to be administered. Inducing this phase shift in the patient will provide a treatment time window on at least the required number of days for the cancer treatment.

In some embodiments, each determined treatment time window is a time window during or after the initiation of the desired phase shift corresponding to, or associated with, a time period at or during which the maximum rate of cancerous tissue cell division is to occur and the maximum rate of healthy tissue cell division is not to occur.

In some embodiments, the processing unit is configured to determine a possible treatment time window for each day after a first day in which the desired phase shift is initiated or is to be initiated up to a number of days after the first day equal to the desired phase shift divided by the shift factor. In these embodiments, the processing unit can be configured to determine a possible treatment time window for each of the days by estimating the timing of the maximum rate of healthy tissue cell division on that day based on the number of days after the first day, an initial timing of the maximum rate of healthy tissue cell division prior to the initiation of the desired phase shift, the desired phase shift and the shift factor; estimating the timing of the maximum rate of cancerous tissue cell division on that day based on the number of days after the first day, an initial timing of the maximum rate of healthy tissue cell division prior to the initiation of the desired phase shift, the desired phase shift, the shift factor and the delay factor; and determining the possible treatment time window for that day as a time window that has a duration that spans the estimated time of the maximum rate of cancerous tissue cell division. In some embodiments, the possible treatment time window ends at or before a midpoint between the estimated time of the maximum rate of healthy tissue cell division and the estimated time of the maximum rate of cancerous tissue cell division. In some embodiments, the time window is centred on the estimated time of the maximum rate of cancerous tissue cell division. In these embodiments, the time window can have a duration that is equal to or less than the duration of the time period between the estimated time of the maximum rate of healthy tissue cell division and the estimated time of the maximum rate of cancerous tissue cell division. The duration of each time window can be dependent on a required amount of time for administering the cancer treatment. In these embodiments, the processing unit can be configured to determine the one or more treatment time windows as the longest ones of the possible treatment time windows, or the ones of the possible treatment time windows having a duration approximately equal to the product of the delay factor and the shift factor.

In some embodiments, the processing unit is further configured to determine a relative timing of the maximum rate of healthy tissue cell division for the patient and the maximum rate of cancerous tissue cell division for the patient, and to determine the one or more treatment time windows if it is determined that the maximum rate of healthy tissue cell division occurs at the same time or at approximately the same time as the maximum rate of cancerous tissue cell division. In these embodiments, the processing unit can be configured to receive one or more measurements of the patient and to determine a relative timing of the maximum rate of healthy tissue cell division for the patient and the maximum rate of cancerous tissue cell division for the patient based on the one or more measurements of the patient. In these embodiments, the one or more measurements can be indicative of the circadian rhythm of the patient and/or indicative of the timing of the maximum rate of healthy tissue cell division for the patient. In these embodiments, the processing unit may be further configured to receive an input indicating a timing of the maximum rate of cancerous tissue cell division.

In some embodiments, the processing unit is further configured to determine the shift factor and/or delay factor for the patient. In some embodiments, the shift factor is patient-specific. In some embodiments, the delay factor is patient-specific and/or dependent on the type of cancer. These embodiments recognise that the treatment time windows can be different for different patients and/or for different types of cancers.

In some embodiments, the treatment time window is determined for a patient for whom the maximum rate of healthy tissue cell division occurs at the same time or at approximately the same time as the maximum rate of cancerous tissue cell division. In some embodiments, the treatment time window is determined for a patient for whom the time difference between the timing of the maximum rate of healthy tissue cell division and the timing of the maximum rate of cancerous tissue cell division is less than a time required to administer a cancer treatment or dose of cancer treatment.

According to a second specific aspect, there is provided a method of determining a treatment time window for administering a cancer treatment to a patient for whom a timing of a maximum rate of healthy tissue cell division occurs is to be shifted with respect to a timing of a maximum rate of cancerous tissue cell division, wherein the timing of the maximum rate of healthy tissue cell division is based on a circadian rhythm of the patient. The method comprises determining one or more treatment time windows for administering the cancer treatment to the patient after the initiation of a desired phase shift in the circadian rhythm based on a shift factor and a delay factor; and provide an output indicating the determined one or more treatment time windows. The shift factor indicates a rate at which the timing of the maximum rate of healthy tissue cell division shifts within a 24-hour cycle in response to the initiation of the desired phase shift. The delay factor indicates a delay from a start of a phase shift in the circadian rhythm of the patient to the start of a corresponding phase shift in the timing of the maximum rate of cancerous tissue cell division. Thus, the method according to the second aspect provides that the timing of one or more treatment time windows in which a cancer treatment can be administered to attack the cancerous cells when they are dividing while minimising the risk of damaging healthy cells when they divide. This can enable the reduction of side effects to the patient (for example caused by damage to healthy tissue by the cancer treatment) and/or an increase in the effectiveness of the cancer treatment (for example if the dosage can be increased in the treatment time window due to the lower risk of unwanted side effects).

In some embodiments, the method further comprises initiating a phase shift in the circadian rhythm according to the desired phase shift. These embodiments have the advantage that, in addition to identifying when one or more treatment time windows might occur following the initiation of a phase shift, the method can include causing the phase shift to occur to create the treatment time windows.

In some embodiments, the desired phase shift is to be caused by the application of a light pattern in the environment of the patient. In these embodiments, the light pattern can be a day-night light pattern. In these embodiments, the light pattern can be a day-night light pattern associated with a different time zone to a time zone at a current location of the patient. In any of these embodiments, the method can further comprise controlling one or more light sources in the environment of the patient to apply the light pattern.

In alternative embodiments, the desired phase shift can be caused by the administration of a medication or substance to the patient. In these embodiments, the method can further comprise controlling a medication dispenser to dispense the medication or substance to the patient to cause the desired phase shift.

In some embodiments, the method can further comprise determining the desired phase shift based on the shift factor and a desired number of days over which the cancer treatment is to be administered to the patient. This has the advantage that the phase shift to be applied to the patient can be customised to the length of the cancer treatment. In these embodiments, the method can comprise determining the desired phase shift as a phase shift that is higher than the product of the shift factor and the desired number of days over which the cancer treatment is to be administered. Inducing this phase shift in the patient will provide a treatment time window on at least the required number of days for the cancer treatment.

In some embodiments, each determined treatment time window is a time window during or after the initiation of the desired phase shift corresponding to, or associated with, a time period at or during which the maximum rate of cancerous tissue cell division is to occur and the maximum rate of healthy tissue cell division is not to occur.

In some embodiments, the method comprises determining a possible treatment time window for each day after a first day in which the desired phase shift is initiated or is to be initiated up to a number of days after the first day equal to the desired phase shift divided by the shift factor. In these embodiments, the method can comprise determining a possible treatment time window for each of the days by estimating the timing of the maximum rate of healthy tissue cell division on that day based on the number of days after the first day, an initial timing of the maximum rate of healthy tissue cell division prior to the initiation of the desired phase shift, the desired phase shift and the shift factor; estimating the timing of the maximum rate of cancerous tissue cell division on that day based on the number of days after the first day, an initial timing of the maximum rate of healthy tissue cell division prior to the initiation of the desired phase shift, the desired phase shift, the shift factor and the delay factor; and determining the possible treatment time window for that day as a time window that has a duration that spans the estimated time of the maximum rate of cancerous tissue cell division. In some embodiments, the possible treatment time window ends at or before a midpoint between the estimated time of the maximum rate of healthy tissue cell division and the estimated time of the maximum rate of cancerous tissue cell division. In some embodiments, the time window is centred on the estimated time of the maximum rate of cancerous tissue cell division. In these embodiments, the time window can have a duration that is equal to or less than the duration of the time period between the estimated time of the maximum rate of healthy tissue cell division and the estimated time of the maximum rate of cancerous tissue cell division. The duration of each time window can be dependent on a required amount of time for administering the cancer treatment. In these embodiments, the method can comprise determining the one or more treatment time windows as the longest ones of the possible treatment time windows, or the ones of the possible treatment time windows having a duration approximately equal to the product of the delay factor and the shift factor.

In some embodiments, the method further comprises determining a relative timing of the maximum rate of healthy tissue cell division for the patient and the maximum rate of cancerous tissue cell division for the patient, and determining the one or more treatment time windows if it is determined that the maximum rate of healthy tissue cell division occurs at the same time or at approximately the same time as the maximum rate of cancerous tissue cell division. In these embodiments, the method can further comprise receiving one or more measurements of the patient and determining a relative timing of the maximum rate of healthy tissue cell division for the patient and the maximum rate of cancerous tissue cell division for the patient based on the one or more measurements of the patient. In these embodiments, the one or more measurements can be indicative of the circadian rhythm of the patient and/or indicative of the timing of the maximum rate of healthy tissue cell division for the patient. In these embodiments, the method may further comprise receiving an input indicating a timing of the maximum rate of cancerous tissue cell division.

In some embodiments, the method further comprises determining the shift factor and/or delay factor for the patient. In some embodiments, the shift factor is patient-specific. In some embodiments, the delay factor is patient-specific and/or dependent on the type of cancer. These embodiments recognise that the treatment time windows can be different for different patients and/or for different types of cancers.

In some embodiments, the treatment time window is determined for a patient for whom the maximum rate of healthy tissue cell division occurs at the same time or at approximately the same time as the maximum rate of cancerous tissue cell division. In some embodiments, the treatment time window is determined for a patient for whom the time difference between the timing of the maximum rate of healthy tissue cell division and the timing of the maximum rate of cancerous tissue cell division is less than a time required to administer a cancer treatment or dose of cancer treatment.

According to a third aspect, there is provided a computer program product comprising a computer readable medium having computer readable code embodied therein, the computer readable code being configured such that, on execution by a suitable computer or processor, the computer or processor is caused to perform the method according to the second aspect or any embodiment thereof.

According to a fourth aspect, there is provided a method of treating cancer in a patient for whom the timing of a maximum rate of healthy tissue cell division is to be shifted with respect to the timing of a maximum rate of cancerous tissue cell division, wherein the timing of the healthy tissue cell division is based on a circadian rhythm of the patient. The method comprises initiating a desired phase shift in the circadian rhythm of the patient; and administering a cancer treatment in one or more treatment time windows after the initiation of the desired phase shift. The one or more treatment time windows are determined according to the method according to the second aspect or any embodiment thereof.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1(a) is a diagram illustrating an alignment between healthy tissue cell division and cancerous tissue cell division, and Fig. 1(b) is a diagram illustrating the principle of a phase shift in the circadian rhythm creating treatment time windows;

Fig. 2(a) is a graph representing an exemplary rate of healthy tissue cell division and cancerous tissue cell division as shown in Fig. 1(a), and Fig. 2(b) is a graph representing an exemplary treatment time window on day 4 of Fig. 1(b);

Fig. 3 is a block diagram of an exemplary apparatus according to an embodiment of the invention;

Fig. 4 is a table illustrating the effect of an exemplary phase shift in the circadian rhythm of a patient and the treatment time windows created; and

Fig. 5 is a flow chart illustrating a method according to an exemplary embodiment.

DETAILED DESCRIPTION OF EMBODIMENTS

As noted above, it has been recognised that a phase shift in the circadian rhythm of the patient shifts the timing of the healthy tissue cell division, but the timing of the cancerous tissue cell division shifts more slowly than the shift in the timing of the healthy tissue cell division. It is generally assumed that the timing of the cancerous tissue cell division shifts at the same rate as the timing of the healthy tissue cell division (for example they both adjust by the same number of hours per day), but there is a delay in the timing of the cancerous tissue cell division starting to shift once the timing of the healthy tissue cell division starts to shift. This lag effectively creates a treatment window for the patient some time following the start of the circadian rhythm phase shift where the cancerous tissue cell division is not aligned with the healthy tissue cell division. A cancer treatment that targets the cell division process and/or is most effective when the cancer cells are dividing can be administered during this treatment window.

In some embodiments, a stimulus (e.g. a light pattern or a circadian rhythm-altering medication) is provided to the patient to shift the circadian rhythm of the patient by the desired amount in order to create the treatment time windows over the course of one or more days.

The lag mentioned above is also referred to as a "differential time shift between healthy and tumour tissue", and is where a phase shift in the circadian cycle is induced and there is one or more periods of time that occur during the transition from the pre-existing phase of the circadian rhythm to the new, phase-shifted, circadian rhythm where the timings of the healthy tissue cell division and cancerous tissue cell division differ. These period(s) of time can provide a treatment window for administering the cancer treatment that span the maximum rate of cancerous tissue cell division.

It will be appreciated that cell division does not just occur at a specific time point in the circadian rhythm of the patient, but instead the rate of cell division (i.e. the number of cells dividing at a given time point/window) typically follows a bell-curve or normal distribution over a 24-hour period. This means that the rate of cell division varies over the 24-hour period with a maximum or peak in the rate at some time point, and lower rates of cell division at other time points. In a static case (i.e. where there is no ongoing adjustment of the phase of the circadian rhythm of the patient) the circadian rhythm of the patient determines or influences the time in the 24-hour cycle at which the maximum or peak occurs for both healthy and cancerous tissue.

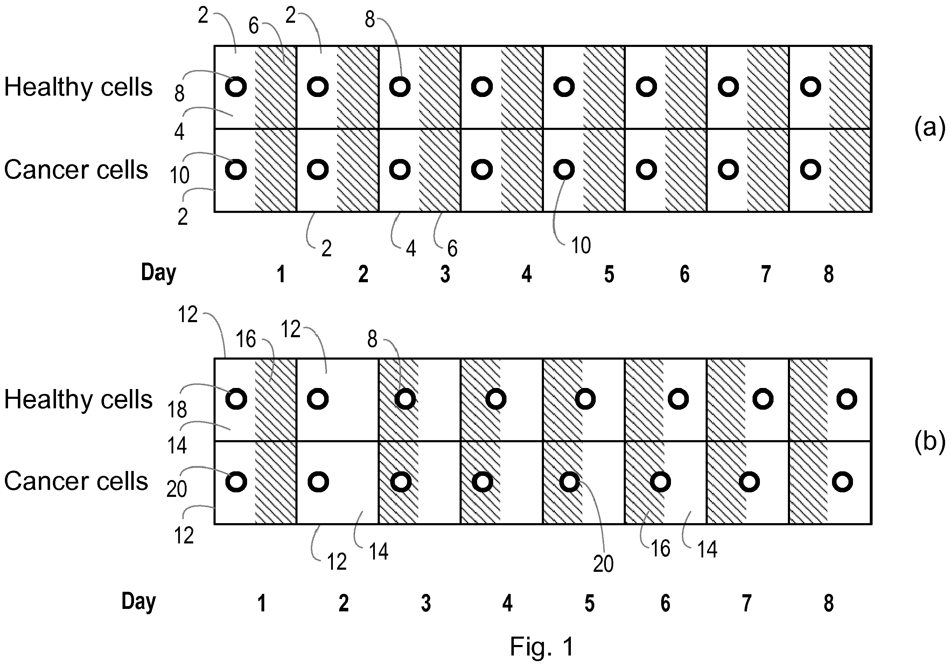

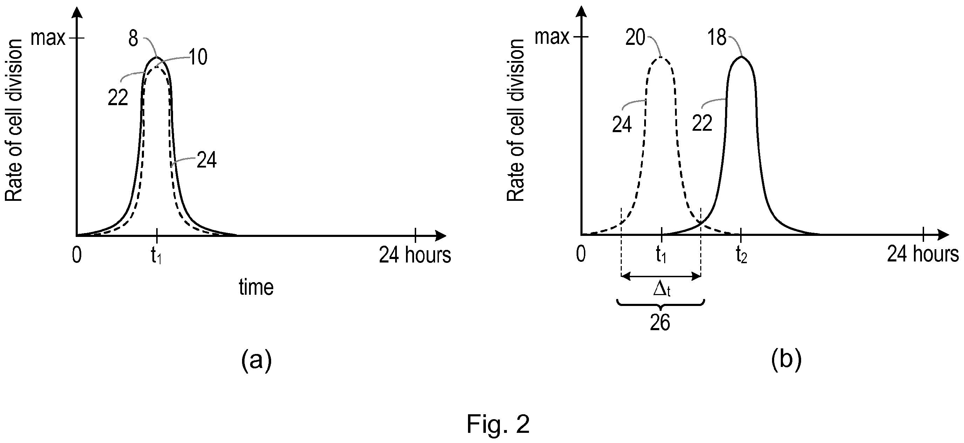

The terms "timing of the healthy tissue cell division" and "timing of the cancerous tissue cell division" used herein refer generally to the point in a 24-hour cycle where the maximum or peak rate of the healthy/cancerous tissue cell division occurs, but as above it is appreciated that cell division also occurs around these time points. Inducing a phase shift in the circadian rhythm causes a shift in the time point in the 24-hour cycle where the maximum or peak rate of the healthy/cancerous tissue cell division occurs. This is illustrated in Figs. 1 and 2.

Fig. 1(a) shows an exemplary series of 24-hour cycles (illustrated by boxes 2 and labelled Day 1, 2, 3, etc.) with a general day-night pattern shown in each box 2. Day (i.e. daylight or day time) is represented by the non-shaded part 4 of each box 2 and night (i.e. night time) is represented by the shaded part 6 of each box 2. Each box 2 in the top row of boxes 2 in Fig. 1(a) is used to illustrate the timing of the healthy tissue cell division in days 1-8, with the time point corresponding to the maximum rate of healthy tissue cell division in each 24-hour cycle (i.e. in each box 2) being represented by a "o" (and labelled 8). Each box 2 in the bottom row of boxes 2 in Fig. 1(a) is used to illustrate the timing of the cancerous tissue cell division in days 1-8, with the time point corresponding to the maximum rate of cancerous tissue cell division in each 24-hour cycle (i.e. in each box 2) being represented by a "o" (and labelled 10). Since the timing of the healthy tissue cell division depends on the circadian rhythm of the patient, the time points 8 representing the timing of the maximum rate of healthy tissue cell division can also be understood as markers or indicators of the phase of the circadian rhythm.

In Fig. 1(a) the day-night pattern does not vary across days 1-8 and thus there is no phase shift in the circadian rhythm of the patient. This means that the circadian rhythm is in a steady state, and the maximum rate of healthy tissue cell division and maximum rate of cancerous tissue cell division occur at the same time in the 24-hour cycle on each day (and at generally the same time as each other). Merely as an example, the timing of the maximum rate of cell division for both healthy tissue and cancerous tissue is shown in Fig. 1(a) as occurring around the mid-point of the day part 2 of each 24-hour cycle.

Fig. 2(a) is a graph representing an exemplary rate of healthy tissue cell division and cancerous tissue cell division according to the alignment shown in Fig. 1(a). As noted above the rate of cell division for both the healthy tissue (represented by curve 22) and the cancerous tissue (represented by curve 24) follows a bell-curve distribution and is at a maximum at a time $t_1$ (corresponding to time points 8 and time points 10 in Fig. 1(a)).

Figure 1(b) shows another exemplary series of 24-hour cycles (illustrated by boxes 12 and labelled Day 1, 2, 3, etc.) with a general day-night pattern shown in each box 12, although rather than illustrate a 'steady state' as in Fig. 1(a), Fig. 1(b) illustrates the effect of causing a phase shift in the circadian rhythm of the patient. As in Fig. 1(a), day (i.e. daylight or day time) is represented by the non-shaded part 14 of each box 12 and night (i.e. night time) is represented by the shaded part 16 of each box 12. Each box 12 in the top row of boxes 12 in Fig. 1(b) is used to illustrate the timing of the healthy tissue cell division in days 1-8, with the time point corresponding to the maximum rate of healthy tissue cell division in each 24-hour cycle (i.e. in each box 12) being represented by a "o" (and labelled 18). Each box 12 in the bottom row of boxes 12 in Fig. 1(b) is used to illustrate the timing of the cancerous tissue cell division in days 1-8, with the time point corresponding to the maximum rate of cancerous tissue cell division in each 24-hour cycle (i.e. in each box 12) being represented by a "o" (and labelled 20). Merely as an example, the initial timing of the maximum rate of cell division for both healthy tissue and cancerous tissue (i.e. the timing on day 1) is shown in Fig. 1(b) as occurring around the mid-point of the day part 12 of each 24-hour cycle. Since the timing of the healthy tissue cell division depends on the circadian rhythm of the patient, the time points 18 representing the timing of the maximum rate of healthy tissue cell division can also be understood as markers or indicators of the phase of the circadian rhythm.

On day 2 a 12-hour phase shift in the circadian rhythm of the patient is initiated by applying a light pattern so that it is 'day' for the whole 24-hour period, with the day-night pattern for days 3-8 being reversed compared to the day-night pattern on day 1. This change in the day-night pattern from day 2 causes the phase of the circadian rhythm of the patient, and thus the timing of the healthy tissue cell division (as indicated by time points 18), to start to shift to align with the new day-night pattern on days 3-8 (i.e. to ultimately align such that the healthy tissue cell division occurs around the mid-point of the day part 12 of each 24-hour cycle).

From day 3 (i.e. the day after the phase shift is initiated), it can be seen that the timing of the healthy tissue cell division in the 24-hour cycle (i.e. the position of time point 18 within each box 12) changes each day relative to the timing of the healthy tissue cell division in the previous day, until the healthy tissue cell division occurs at the right time point in the 24-hour period (i.e. around the mid-point of the day part 12 of each 24-hour cycle). The rate at which the phase of the circadian rhythm shifts in response to the altered lighting pattern (and thus the rate at which the timing of the healthy tissue cell division shifts in response to the altered lighting pattern) typically depends on the patient, but as an example, the timing/phase may shift by 2 hours per day. In this example, the circadian rhythm/healthy tissue cell division phase shift of 12 hours is complete on day 8, six days after the phase started to shift.

As noted above, the timing of the cancerous tissue cell division is dependent on the circadian rhythm and/or timing of the healthy tissue cell division, but there is a lag between a shift in the circadian rhythm/timing of the healthy tissue cell division and a corresponding shift in the timing of the cancerous tissue cell division. This can be seen in the bottom row of boxes 12 in Fig. 1(b), where, following the timing of the healthy tissue cell division starting to shift from day 3, the timing of the cancerous tissue cell division only starts to shift on day 5 (i.e. here the lag is two days).

Comparing the top and bottom rows of boxes 12 in Fig. 1(b) shows that this lag leads to the relative timings of the healthy tissue cell division and cancerous tissue cell division being different on days 3-8. Therefore, there is an opportunity on days 3-8 to administer a cancer treatment to target the cancerous cells during their maximum rate of cell division while the healthy cells are not at their maximum rate of cell division. Indeed, with the exemplary 2 hours/day shift in the timing of the healthy tissue cell division and a lag of two days in a corresponding shift in the timing of the cancerous tissue cell division, this creates a 4-hour gap between the timing of the healthy tissue cell division and the timing of the cancerous tissue cell division on days 4-8, and a 2-hour gap on days 3 and 9 (day 9 is not shown in Fig. 1). Fig. 2(b) is a graph representing an exemplary treatment time window on day 4 of Fig. 1(b). Thus, Fig. 2(b) shows that the rate of healthy tissue cell division (represented by curve 22) has shifted so that the maximum rate of healthy tissue cell division is at a time $t_2$, while the rate of cancerous tissue cell division (represented by curve 24) has not yet started to adapt or shift, and so the maximum rate of cancerous tissue cell division is still at time $t_1$. It can be seen in Fig. 2(b) that there is a gap between the maximum rates and this provides a treatment time window in which a cancer treatment can be administered. In particular, for a cancer treatment that is most effective when cancer cells are dividing, the treatment time window can be determined that spans the maximum rate of cancerous tissue cell division (i.e. time $t_1$). As an example, a treatment time window can end at or before the midpoint between the times that the maximum rates occur (i.e. the end of the treatment time window can be at or before a time $[t_1 + ((t_2 - t_1) / 2)]$), with the treatment time window having a duration that spans the estimated time of the maximum rate of cancerous tissue cell division (i.e. the treatment time window includes the time at which the maximum rate of cancerous tissue cell division occurs). In some embodiments, the treatment time window can be centred on the estimated time of the maximum rate of cancerous tissue cell division, and the duration of the treatment time window will be equal to less than half of the time difference between the times that the maximum rates occur (i.e. the maximum duration is $t_2 - t_1$). Assuming that the distributions 22, 24 follow generally the same pattern, the midpoint between the times that the maximum rates occur will correspond to the time where the likelihood of healthy tissue cell division is approximately the same as the likelihood of cancerous tissue cell division (with healthy tissue cell division being more likely than cancerous tissue cell division one side of the midpoint and cancerous tissue cell division being more likely than healthy tissue cell division on the other side of the midpoint). In some cases the particular duration can depend on the time required to administer the cancer treatment.

Fig. 2(b) shows an exemplary treatment time window 26 that is centred on the maximum rate of cancerous tissue cell division (i.e. time $t_1$) and the window 26 has a duration that is equal to the time difference between the times that the maximum rates occur (i.e. $\Delta t = t_2 - t_1$).

Thus, the invention provides that, given a shift in the phase of the circadian rhythm that is be induced in a patient (or that is already being induced in the patient), one or more treatment time windows can be determined where the healthy tissue cell division is not aligned (or is not fully aligned, given the normal distribution typically associated with cell division). Some embodiments provide that the required phase shift (also referred to herein as the "desired phase shift") can be determined. This may be the case where it is desirable to provide a treatment time window over a particular number of days.

In the following description of the invention, the rate at which the timing of the healthy tissue cell division shifts in response to a phase shift is referred to herein as the "shift factor", X, and is typically expressed in hours/day. The value of the shift factor may depend on the specific patient. The lag between a shift in the timing of the healthy tissue cell division and a corresponding shift in the timing of the cancerous tissue cell division is referred to herein as the "delay factor", Y, and is typically expressed as a number of days. The value of the delay factor may depend on the specific patient and/or may depend on the specific type of cancer tissue.

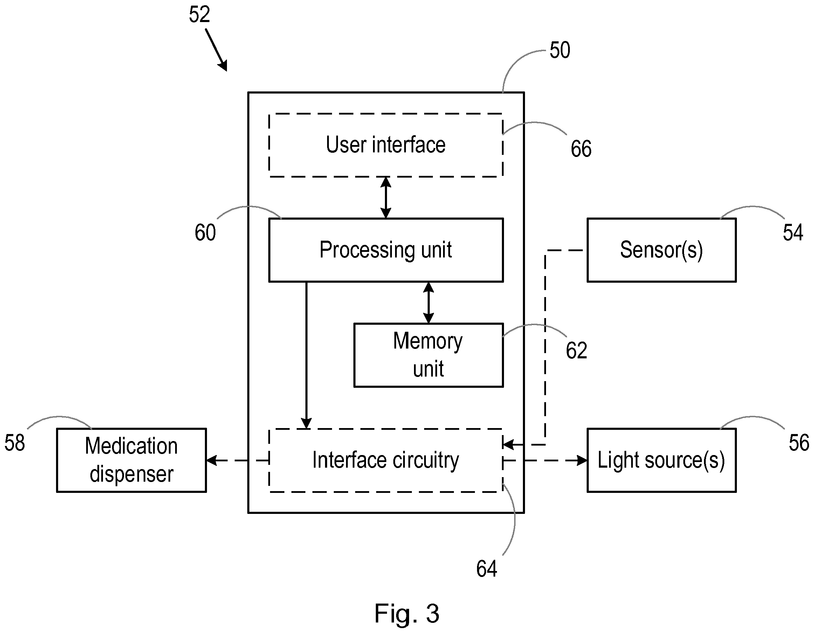

A block diagram of an exemplary apparatus 50 that can be used to determine a treatment time window for administering a cancer treatment to a patient in accordance with the techniques described herein is shown in Fig. 3. The apparatus 50 is shown as part of a system 52 that can include one or more of: one or more sensors 54 for monitoring the patient, for example to enable the apparatus 50 (or another device) to measure or estimate the circadian rhythm of the patient; one or more light sources 56 that can be controlled by the apparatus 50 (or another device) to induce a desired phase shift in the circadian rhythm of the patient; and a medication dispenser 58 that can be controlled by the apparatus 50 (or another device) to dispense a medication or substance (e.g. melatonin) that can induce a desired phase shift in the circadian rhythm of the patient.

The apparatus 50 includes a processing unit 60 that controls the operation of the apparatus 50 and that can be configured to execute or perform the methods described herein. The processing unit 60 can be implemented in numerous ways, with software and/or hardware, to perform the various functions described herein. The processing unit 60 may comprise one or more microprocessors or digital signal processor (DSPs) that may be programmed using software or computer program code to perform the required functions and/or to control components of the processing unit 60 to effect the required functions. The processing unit 60 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 60 is connected to a memory unit 62 that can store data, information and/or signals for use by the processing unit 60 in controlling the operation of the apparatus 50 and/or in executing or performing the methods described herein. In some implementations the memory unit 62 stores computer-readable code that can be executed by the processing unit 60 so that the processing unit 60 performs one or more functions, including the methods described herein. The memory unit 62 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The apparatus 50 may also include interface circuitry 64 for enabling a data connection to and/or data exchange with other devices, including any one or more of servers, databases, user devices, and sensors (e.g. sensor(s) 54). The connection may be direct or indirect (e.g. via the Internet), and thus the interface circuitry 64 can enable a connection between the apparatus 50 and a network, such as the Internet, via any desirable wired or wireless communication protocol. For example, the interface circuitry 64 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 64 (and thus apparatus 50) may include one or more suitable antennas for transmitting/receiving over a transmission medium (e.g. the air). Alternatively, in the case of a wireless connection, the interface circuitry 64 may include means (e.g. a connector or plug) to enable the interface circuitry 64 to be connected to one or more suitable antennas external to the apparatus 50 for transmitting/receiving over a transmission medium (e.g. the air). If present in the apparatus 50, the interface circuitry 64 is connected to the processing unit 60.

In some embodiments, the apparatus 50 comprises a user interface 66 that includes one or more components that enables a user of apparatus 50 to input information, data and/or commands into the apparatus 50, and/or enables the apparatus 50 to output information or data to the user of the apparatus 50. For example, the user interface 66 can be used to output information on the treatment time windows determined according to the techniques described herein. The user interface 66 can comprise any suitable input component(s), including but not limited to a keyboard, keypad, one or more buttons, switches or dials, a mouse, a track pad, a touchscreen, a stylus, a camera, a microphone, etc., and the user interface 66 can comprise any suitable output component(s), including but not limited to a display screen, one or more lights or light elements, one or more loudspeakers, a vibrating element, etc.

The apparatus 50 can be any type of electronic device or computing device. For example the apparatus 50 can be, or be part of, a server, a computer, a laptop, a tablet, a smartphone, etc.

It will be appreciated that a practical implementation of an apparatus 50 may include additional components to those shown in Fig. 3. For example the apparatus 50 may also include a power supply, such as a battery, or components for enabling the apparatus 50 to be connected to a mains power supply.

As noted above, the sensor(s) 54 can be used for monitoring the patient, for example to enable the apparatus 50 (or another device) to measure or estimate the circadian rhythm of the patient. The sensor(s) 54 may be separate from the apparatus 50 and can be part of a device that is carried or worn by the patient. In that case, the sensor(s) 54 may be connected or connectable (e.g. via a wireless connection) to the apparatus 50 (and in particular the interface circuitry 64) in order for the measurements from the sensor(s) 54, or the results of processing of the measurements from the sensor(s) 54 (e.g. an indication of the circadian rhythm of the patient), to be provided to the apparatus 50/processing unit 60. In these implementations, the sensor(s) 54 may be part of a watch, smartwatch or bracelet that is to be worn on the wrist, although those skilled in the art will appreciate that other form factors can be used.

In alternative implementations, the sensor(s) 54 can be part of the apparatus 50, and the sensor(s) 54 can be connected to the processing unit 60 so that the measurements from the sensor(s) 54 can be provided to the processing unit 60. In these implementations, the apparatus 50 can be in the form of a watch, smartwatch or bracelet that is to be worn on the wrist, although those skilled in the art will appreciate that other form factors can be used.

The sensor(s) 54 can measure any parameters that can be used to determine or estimate the circadian rhythm of the patient. The parameters can include one or more physiological characteristics of the patient, such as temperature changes over a circadian period (in which case the sensor(s) 54 can be or include a temperature sensor), or the hormonal cortisol level variation during a circadian period. The parameters can also or alternatively include one or more environmental parameters, such as the light-dark cycle to which the patient is exposed.

One indicator of the circadian rhythm of a patient, and in particular an indicator of when cell division is occurring, is the occurrence of the lowest core body temperature in the 24-hour cycle. Techniques for the processing or analysis of sensor measurements to determine or estimate the circadian rhythm of a patient are known in the art, and further details are not provided herein.

As noted above, the system 52 can include one or more light sources 56 that can be controlled by the apparatus 50 or another device to induce a desired phase shift in the circadian rhythm of the patient. It is known that changes in a regular 24-hour based day-night light pattern will lead to changes in the circadian rhythm of a patient. Based on a desired phase shift that is to be induced in the circadian rhythm, the light source(s) 56 can be controlled to provide a required light pattern to induce that phase shift. As such, the light pattern may be a pattern that spans several days (e.g. as shown in Fig. 1(b)).

The light source(s) 56 may be any type or combination of types of light source 56 that can emit light. For example the light source(s) 56 can be one or more light emitting diodes, LEDs, fluorescent bulbs, or incandescent bulbs, although other types of light source can be used. In some embodiments, the light source(s) 56 can comprise, or be, light sources having different colours (for example red, green and blue LEDs) that can be controlled individually or together to provide light of a desired colour.

The light source(s) 56 are typically located in the environment of the patient, and for example can include any one or more of room lighting, ceiling lighting, table lamps, etc. The light source(s) 56 may be located in a dedicated treatment room in which the patient is to stay for several days during the cancer treatment process. Such a treatment room could be provided in a hospital or other healthcare facility, and could be used for multiple patients at a time.

Where the light source(s) 56 are controlled by the apparatus 50, the light source(s) 56 can be connected to the processing unit 60 via the interface circuitry 64 to enable control signals to be communicated from the processing unit 60 to the light source(s) 56.

The light source(s) 56 may be such that one or more parameters of the light emitted by the light source(s) 56 are controllable. For example the intensity (or maximum intensity) of the light emitted by the light source(s) 56 may be controllable, the colour of the light emitted by the light source(s) 56 may be controllable, the light source(s) 56 may be controllable to emit pulsating light, with the frequency of the pulsation being controllable, and/or the duration of each pulse of light from the light source(s) 56 may be controllable.

As noted above, the system 52 can include a medication dispenser 58 that can be controlled by the apparatus 50 or another device to dispense a medication or substance. It is known that certain medications or substances can induce a phase shift in the circadian rhythm of a patient. One such medication/substance is melatonin, and those skilled in the art will be aware of other medications or substances that can be used. Where the medication dispenser 58 is controlled by the apparatus 50, the medication dispenser 58 can be connected to the processing unit 60 via the interface circuitry 64 to enable control signals to be communicated from the processing unit 60 to the medication dispenser 58. In some cases the medication dispenser 58 can also or alternatively be used to dispense the cancer treatment (e.g. chemotherapy tablets) at appropriate times, e.g. according to the determined treatment time windows.

Briefly, the invention provides that one or more treatment time windows for administering a cancer treatment to a patient are determined. The treatment time windows can be determined for a patient for whom the maximum rate of healthy tissue cell division occurs at the same time or at approximately the same time as the maximum rate of cancerous tissue cell division, and/or they can be determined for a patient for whom the time difference between the timing of the maximum rate of healthy tissue cell division and the timing of the maximum rate of cancerous tissue cell division is less than a time required to administer a cancer treatment or dose of cancer treatment (e.g. a radiotherapy session). The treatment time windows will be after the initiation of a desired phase shift in the circadian rhythm of the patient, and the treatment time windows are determined based on a shift factor and delay factor. As noted above, the shift factor indicates a rate at which the timing of the maximum rate of healthy tissue cell division shifts within a 24-hour cycle in response to the initiation of the desired phase shift, and the delay factor indicates a delay from a start of a phase shift in the circadian rhythm of the patient to the start of a corresponding phase shift in the timing of the maximum rate of cancerous tissue cell division. An indication of the determined treatment time windows is output to a user of the apparatus 50. The user of the apparatus 50 may be a healthcare professional, a care provider and/or the patient themselves. The indication may be an indication of the time at which each determined treatment time window starts and/or ends. Alternatively or in addition, the indication may be output at a time corresponding to the start time of a treatment time window, with the indication indicating that a cancer treatment can be administered or started.

Various exemplary details of the invention are provided below for embodiments where the desired phase shift is achieved using a light pattern, but it will be appreciated that, unless otherwise indicated, the exemplary details are also applicable to embodiments that achieve the desired phase shift using a medication or substance, or using any other suitable mechanism.

In some embodiments, the current phase of the circadian rhythm of cell division in the patient is determined for healthy tissue, and optionally also cancerous tissue (although alternatively it can be assumed that the cell division of the cancerous tissue is aligned with the cell division of the healthy tissue). The circadian rhythm of the healthy tissue can be determined using measurements from the sensor(s) 54 as noted above. Alternatively, a user of the apparatus 50 can manually input an indication of the circadian rhythm of the patient or an indication of the timing of the maximum rate of healthy tissue cell division. If required, the circadian rhythm of the cancerous tissue can be determined by the apparatus 50 using specialist methods, which includes invasive collection of cancerous body cells and then offline analysis (for example in a laboratory, which can take at least 24 hours. Alternatively, a user of the apparatus 50 can manually input an indication of the timing of the maximum rate of cancerous tissue cell division.

If the maximum rates of the healthy tissue cell division and the cancerous tissue cell division occur at different times (i.e. at different points of the circadian rhythm), then there may already be a suitable treatment time window for administering the cancer treatment (e.g. at or around the time of maximum cancerous tissue cell division), and it may not be necessary to create a treatment time window by causing a phase shift in the circadian rhythm of the patient. However, it will be appreciated that it may be desirable to temporarily increase the size of the treatment time window by inducing the phase shift in the circadian rhythm of the patient, in which case the techniques described herein can still be used.

In the following description, it is assumed that the maximum rates of the healthy tissue cell division and the cancerous tissue cell division occur at the same time or substantially the same time, which in fact means that the peaks of the normal distribution are not farther apart than a predefined shift (for example one sigma of the normal distribution).

Due to the (non-zero) delay factor Y, a shift in the light-dark regime in the environment of the patient will disrupt the circadian rhythm of healthy cells and tumour cells in different ways. The light pattern will change the circadian stage of healthy cells directly by X hours per day (the shift factor), while cancerous cells will follow the shift by the healthy cells with a delay of Y days. The shift of the circadian stage of the healthy cells and delayed shift of the cancerous cells creates a treatment time window in which there is no simultaneous cell division (e.g. at the lowest core body temperature in a 24-hour cycle) of cancerous and healthy cells. The size of each treatment time window depends on X and Y, and X and/or Y can be different per patient and/or according to the type of cancerous tissue involved.

The maximum length (duration) of a treatment time window can be given by the product of the shift factor and the delay factor (i.e. X*Y). Treatment time windows having the maximum duration will occur from Y days after the start of the phase shift in the healthy tissue cell division and occur for a number of days equal to the total (desired) phase shift divided by the shift factor, X (i.e. the number of days that it takes for the healthy tissue cell division to adapt to the phase shifted circadian rhythm).

Given the rhythm of healthy tissue cell division and given the moments of cancerous tissue cell division, the apparatus 50 can compute or determine a light pattern to be applied to the patient that will induce a shift in the patient's circadian rhythm that is sufficient to induce a shift in the timing of the healthy tissue cell division and create the treatment time window due to the delay factor. The light pattern may also be computed or determined based on requiring a cancer treatment to be administered over the course of several days. For example, if a cancer treatment should be administered each day for three days, the light pattern can be determined such that the phase shift is large enough to create a treatment time window for at least three days. The magnitude of the phase shift required to achieve this is dependent on the shift factor X for the patient.

It will be appreciated that the phase shift induced in the patient does not need to be static, but a 'continuous' or 'dynamic' phase shift could be induced in the patient where it is desired to increase the number of days in which a treatment time window exists. For example, in some embodiments, a 'static' phase shift can be induced where a light pattern is applied on one day that is maintained for subsequent days to cause a particular phase shift over the course of several days (e.g. as shown in Fig. 1(b)). In other embodiments, the light pattern can be changed over time so that it is continuously out of phase with the circadian rhythm, meaning that the circadian rhythm is not able to fully 'catch up', and instead each day the circadian rhythm continues to shift in response to the changing light pattern each day. For example, on day 1 a light pattern with a 4-hour phase shift can be applied in the environment of the patient. On day 2, a light pattern with a further 2-hour phase shift can be applied. On day 3, a further 2-hour phase shift can be applied, and so on until the required number of days with treatment time windows have been created (and used to administer a cancer treatment).

It will be appreciated that the phase shift induced by the light patterns discussed above, can also occur in response to the patient moving time zones (particularly when flying long distances). Thus, in some embodiments, the apparatus 50 can be used to determine treatment time windows for a patient that is to move time zones (for example if they are going on or returning from a holiday), and to output indications of treatment time windows following their flight.

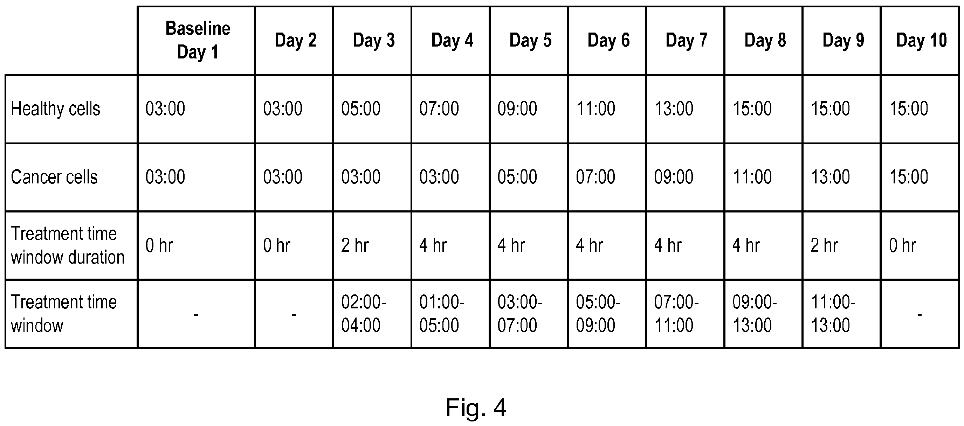

The table in Fig. 4 illustrates exemplary treatment time windows that can be created by a 12-hour phase shift in the circadian rhythm of a patient, and determined by the apparatus 50. In this exemplary illustration, the treatment time windows are centred on the time of the maximum rate of cancerous tissue cell division, and they have a duration equal to the difference between the times at which the maximum rates occur. The example shown in Fig. 4 is similar to the example shown in Fig. 1 and thus, for this exemplary patient, the shift factor X is 2 hours/day and the delay factor Y is 2 days. Each column in the table relates to a day, with Day 1 showing the baseline, and day 2 being the day on which the 12-hour phase shift is initiated (e.g. by the light-dark pattern in the environment of the patient being reversed compared to the current (e.g. natural) light-dark pattern as in Fig. 1(b), or by any other suitable light pattern, e.g. one in which the light-dark pattern is shifted by a number of hours per day up to a maximum of 12 hours after several days). The first line of the table indicates the timing of the maximum rate of healthy tissue cell division on each day, the second line of the table relates to the timing of the maximum rate of cancerous tissue cell division on each day, the third line of the table indicates the duration of a treatment time window on each day, and the fourth line of the table indicates the start and end times of the treatment time window on each day.

Thus, it can be seen that on day 1 the maximum rate of healthy tissue cell division and cancerous tissue cell division occurs at 03:00. Following the initiation of the phase shift on day 2, the timing of the maximum rate of healthy tissue cell division starts to shift so that on day 3, the maximum rate of healthy tissue cell division occurs at 05:00 (due to X being 2 hours/day). The delay factor of 2 days means that the maximum rate of cancerous tissue cell division remains at 03:00 meaning that there is a 2-hour long treatment time window on day 3 that is between 02:00 and 04:00. On day 4 the maximum rate of healthy tissue cell division occurs at 07:00 while the maximum rate of cancerous tissue cell division remains at 03:00 meaning that there is a 4-hour long treatment time window that is between 01:00 and 05:00. From day 5 to day 8, the timing of the maximum rate of cancerous tissue cell division shifts by 2 hours/day (the same as the shift factor X for the healthy tissue cell division), while the timing of the maximum rate of healthy tissue cell division continues to shift by 2 hours/day, maintaining the 4-hour long treatment time window. The timing of the maximum rate of healthy tissue cell division achieves the 12-hour phase shift from the baseline of 03:00 on day 8, and on day 9 the treatment time window is only 2 hours long as the timing of the maximum rate of cancerous tissue cell division 'catches up' with the timing of the maximum rate of healthy tissue cell division. On day 10 both the healthy tissue cell division and cancerous tissue cell division are aligned again, and there is no suitable treatment time window for administering a cancer treatment.

As noted above, rather than apply a 'static' phase shift as shown in Fig. 4, a 'dynamic' or 'continuous' phase shift can be used to extend the number of days in which a treatment time window occurs. For example, on day 8 where the circadian rhythm is aligned with the 12-hour phase-shifted light pattern (or other phase shift inducing mechanism, such as melatonin tablets), the light pattern (or melatonin tablet dosage/timing) can be further shifted (e.g. by another 12 hours), so that the circadian rhythm continues to adapt from day 9 onwards. This would double the number of days where a 4-hour treatment time window exists, and also has the advantage that the patient is 'returned' to their original day-night pattern at the end of the treatment programme. An alternative dynamic phase shift could change the light pattern (or other mechanism) by a certain amount each day (e.g. the light pattern could be phase shifted by at least X hours/day) so that the circadian rhythm is required to adapt each day that the phase shift continues to change. This type of dynamic phase shift would not only enable treatment time windows to be provided on any number of desired days (provided the phase shift continues to change), but the gradual phase shift each day might also be less disorientating for the patient than a larger phase shift (e.g. 12 hours) occurring on one day.

Based on the table in Fig. 4, the apparatus 50 can output an indication of the determined one or more treatment time windows to a user in the form of the start time of a treatment time window (e.g. 03:00 on day 5). The indication may also include one or both of the duration of the treatment time window (e.g. 4 hours) and the end time of the treatment time window (e.g. 07:00 on day 5). The indication may be directly output to the user by the apparatus 50, for example by displaying the indication on a display component of the user interface 66, and/or by playing an audio message indicating the timing/size of the treatment time window via a loudspeaker component of the user interface 66. Alternatively, the indication may be output in the form of an electronic file, data file or control signal that is provided to a user device, such as a smart phone, tablet, laptop, etc.

Thus, embodiments of this disclosure provide an apparatus 50 that operates as a decision support system that can use the output of one or more sensor(s) 54 (for example in a smartwatch) to determine the current circadian rhythm of the patient, determine a phase shift to be induced in the circadian rhythm of the patient (e.g. to provide a treatment time window of at least a minimum length, or the largest possible treatment time window, and provide a treatment time window on a required number of days), and determine the treatment time windows that will occur as a result of the phase shift being caused. The apparatus 50 can also inform the user (e.g. physician or healthcare provider) about the current phasing of the patient's circadian rhythm, for example once the lighting pattern has been changed, based on the measurements from the sensor(s) 54.

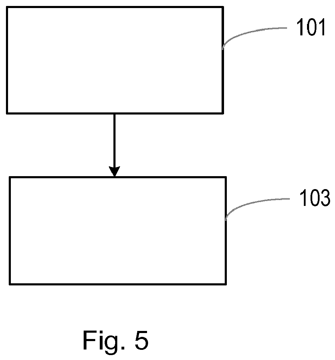

The flow chart in Fig. 5 illustrates an exemplary method according to the techniques described herein. One or more of the steps of the method can be performed by the processing unit 60 in the apparatus 50, in conjunction with any of the memory unit 62, interface circuitry 64 and user interface 66 as appropriate. The processing unit 60 may perform the one or more steps in response to executing computer program code, that can be stored on a computer readable medium, such as, for example, the memory unit 62.

The method in Fig. 5 is directed to determining a treatment time window for administering a cancer treatment to a patient. The method is applicable to a patient for whom a maximum rate of healthy tissue cell division occurs at the same time or at approximately the same time as a maximum rate of cancerous tissue cell division, or for whom the maximum rates occur at different times, but further temporal separation is required (e.g. to increase the duration of the current time gap between the maximum rates if the current separation (time gap) is not sufficient to enable an effective cancer treatment without affecting the healthy tissue).

In step 101, the method comprises determining one or more treatment time windows for administering the cancer treatment to the patient based on a shift factor (X) and a delay factor (Y). These treatment time windows will be determined for one or more days after a desired phase shift in the circadian rhythm of the patient has been initiated.

In some embodiments, step 101 may be performed following a determination that the maximum rate of healthy tissue cell division for the patient occurs at the same time or at approximately the same time as a maximum rate of cancerous tissue cell division. In some embodiments, step 101 may be performed following a determination that the time difference between the timing of the maximum rate of healthy tissue cell division and the timing of the maximum rate of cancerous tissue cell division is less than a time required to administer a cancer treatment or a dose of a cancer treatment (e.g. the time required to complete a radiotherapy session).

In step 103, an output is provided to a user (e.g. physician) indicating the determined one or more treatment time windows. As noted above, this indication can be in the form of a start time, end time and/or duration of one or more of the determined treatment time windows.

The method may further comprise the step of initiating the phase shift in the circadian rhythm of the patient according to the desired phase shift. This can involve controlling (directly or indirectly) one or more light source(s) 56 to change a light pattern in the environment of the patient, or controlling (directly or indirectly) the dispensing of a suitable medication or substance from medication dispenser 58. The light pattern to be applied in the environment of the patient can be a day-night light pattern that is phase-shifted with respect to the day-night light pattern associated with the time zone that the patient is currently in.

In some embodiments, the method can include determining the desired phase shift. The desired phase shift can be determined based on the shift factor and a desired number of days over which the cancer treatment is to be administered to the patient. In particular, the desired phase shift can be determined as a phase shift that is higher than the product of the shift factor and the desired number of days over which the cancer treatment is to be administered. In some cases, there may be a particular day on which a cancer treatment is intended to be administered, and the desired phase shift can be determined so that a suitable treatment time window will occur on the particular day. Other techniques for determining the desired phase shift are possible (for example where a dynamic phase shift is to be used.

In the case of a static phase shift, step 101 can comprise determining a possible treatment time window for each day after a first day in which the desired phase shift is initiated or is to be initiated, up to a number of days after the first day equal to the desired phase shift (expressed in hours) divided by the shift factor X (expressed in hours per day).

In step 101, a possible treatment time window can be determined for each day by estimating the timing of the maximum rate of healthy tissue cell division on that day, estimating the timing of the maximum rate of cancerous tissue cell division on that day and then determining the possible treatment time window for that day as a time window that spans the estimated time of the maximum rate of cancerous tissue cell division. In some embodiments, the possible treatment time window ends at or before a midpoint between the estimated time of the maximum rate of healthy tissue cell division and the estimated time of the maximum rate of cancerous tissue cell division.

The timing of the maximum rate of healthy tissue cell division (denoted $h\_n$) on day n after the initiation of the phase shift (which occurs on day n = 0) can be estimated based on the number of days after day n = 0, an initial timing (denoted h_initial) of the maximum rate of healthy tissue cell division prior to the initiation of the desired phase shift (where h initial can be determined using the sensor(s) 54 or manually input into the apparatus 50), the desired phase shift (denoted p) and the shift factor X. For example, the timing of the maximum rate of healthy tissue cell division on day n can be given by:

$$h\_n = h\_initial + (n * X), \quad \text{if } n < p / X,$$

$$h\_n = h\_initial + p, \quad \text{if } n \geq p / X.$$

The timing of the maximum rate of cancerous tissue cell division (denoted c_n) on day n can be estimated based on the number of days after day n = 0, an initial timing (denoted c_initial) of the maximum rate of healthy tissue cell division prior to the initiation of the desired phase shift (where c_initial can be determined using the sensor(s) 54, manually input into the apparatus 50, or assumed to be equal to h initial as the maximum rates are aligned or substantially overlapping), the desired phase shift p, the shift factor X and the delay factor Y. For example, the timing of the maximum rate of cancerous tissue cell division on day n can be given by:

$$c\_n = c\_initial, \quad \text{if } n \leq Y,$$

$$c\_n = c\_initial + ((n - Y) * X), \quad \text{if } Y < n < (p / X) + Y,$$

$$c\_n = c\_initial + p, \quad \text{if } n \geq (p / X) + Y.$$

A treatment time window for day n can be determined as ending at or before the midpoint between h_n and c_n, with a duration that spans c_n. In some embodiments, the treatment time window is centred on c_n. In that case, the treatment time window can have a maximum duration of $|(h\_n - c\_n)|$.

Step 101 can comprise determining the one or more treatment time windows as the longest ones of the possible treatment time windows (i.e. the treatment time windows for any day where $|(c\_n - h\_n)|$ is a maximum amongst the treatment time windows for all of the days). Alternatively, step 101 can comprise determining the one or more treatment time windows as the ones of the possible treatment time windows having a duration approximately equal to the product of the delay factor and the shift factor (i.e. the treatment time windows for any day where $|(c\_n - h\_n)| = X*Y$).

In some embodiments, the method can determine the shift factor and/or delay factor for the patient, for example by continuously or periodically monitoring the level of hormonal cortisol and/or core body temperature of the patient.

In some embodiments, the method in Fig. 5 can be used as part of a method of treating cancer in a patient. In this method, the desired phase shift in the circadian rhythm of the patient can be initiated (for example by changing the light (e.g. day-night) pattern) and a cancer treatment can be administered in one or more treatment time windows determined according to step 101 after the initiation of the desired phase shift.

There is therefore provided a method, computer program product and apparatus that enable a suitable treatment time window to be derived (and created) for a patient to enable a more effective cancer treatment.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1.  An apparatus for determining a treatment time window for administering a cancer treatment to a patient for whom a timing of a maximum rate of healthy tissue cell division is to be shifted with respect to a timing of a maximum rate of cancerous tissue cell division, wherein the timing of the maximum rate of healthy tissue cell division is based on a circadian rhythm of the patient, the apparatus comprising a processing unit configured to:

determine one or more treatment time windows for administering the cancer treatment to the patient after the initiation of a desired phase shift in the circadian rhythm based on:

- a shift factor indicating a rate at which the timing of the maximum rate of healthy tissue cell division shifts within a 24-hour cycle in response to the initiation of the desired phase shift, and
- a delay factor indicating a delay from a start of a phase shift in the circadian rhythm of the patient to the start of a corresponding phase shift in the timing of the maximum rate of cancerous tissue cell division; and

provide an output indicating the determined one or more treatment time windows.

**2.** An apparatus as claimed in claim 1, wherein the processing unit is further configured to:
initiate a phase shift in the circadian rhythm according to the desired phase shift.

**3.** An apparatus as claimed in claim 1 or 2, wherein the desired phase shift is to be caused by the application of a light pattern in the environment of the patient or by the administration of a medication or substance to the patient.

**4.** An apparatus as claimed in claim 1 or 2, wherein the processing unit is further configured to initiate the phase shift by controlling one or more light sources in the environment of the patient to apply a light pattern in the environment of the patient, or controlling a medication dispenser to dispense a medication or substance to the patient.

**5.** An apparatus as claimed in any of claims 1-4, wherein the processing unit is configured to determine the desired phase shift based on the shift factor and a desired number of days over which the cancer treatment is to be administered to the patient.

**6.** An apparatus as claimed in claim 5, wherein the processing unit is configured to determine the desired phase shift as a phase shift that is higher than the product of the shift factor and the desired number of days over which the cancer treatment is to be administered.

**7.** An apparatus as claimed in any of claims 1-6, wherein the processing unit is configured to determine a possible treatment time window for each day after a first day in which the desired phase shift is initiated or is to be initiated up to a number of days after the first day equal to the desired phase shift divided by the shift factor.

**8.** An apparatus as claimed in claim 7, wherein the processing unit is configured to determine a possible treatment time window for each of the days by:

estimating the timing of the maximum rate of healthy tissue cell division on that day based on the number of days after the first day, an initial timing of the maximum rate of healthy tissue cell division prior to the initiation of the desired phase shift, the desired phase shift and the shift factor;
estimating the timing of the maximum rate of cancerous tissue cell division on that day based on the number of days after the first day, an initial timing of the maximum rate of healthy tissue cell division prior to the initiation of the desired phase shift, the desired phase shift, the shift factor and the delay factor; and
determining the possible treatment time window for that day as a time window that spans the estimated time of the maximum rate of cancerous tissue cell division.

**9.** An apparatus as claimed in claim 8, wherein the processing unit is configured to determine the one or more treatment time windows as the longest ones of the possible treatment time windows, or the ones of the possible treatment time windows having a duration approximately equal to the product of the delay factor and the shift factor.

**10.** A method of determining a treatment time window for administering a cancer treatment to a patient for whom a timing of a maximum rate of healthy tissue cell division is to be shifted with respect to a timing of a maximum rate of cancerous tissue cell division, wherein the timing of the maximum rate of healthy tissue cell division is based on a circadian rhythm of the patient, the method comprising:

determining one or more treatment time windows for administering the cancer treatment to the patient after the initiation of a desired phase shift in the circadian rhythm based on:

- a shift factor indicating a rate at which the timing of the maximum rate of healthy tissue cell division shifts within a 24-hour cycle in response to the initiation of the desired phase shift, and
- a delay factor indicating a delay from a start of a phase shift in the circadian rhythm of the patient to the start of a corresponding phase shift in the timing of the maximum rate of cancerous tissue cell division; and

providing an output indicating the determined one or more treatment time windows.

11. A method as claimed in claim 10, wherein the method further comprises:
initiating a phase shift in the circadian rhythm according to the desired phase shift.

12. A method as claimed in claim 10 or 11, wherein the desired phase shift is to be caused by the application of a light pattern in the environment of the patient or by the administration of a medication or substance to the patient.

13. A method as claimed in claim 10 or 11, wherein the method further comprises initiating the phase shift by controlling one or more light sources in the environment of the patient to apply a light pattern in the environment of the patient, or controlling a medication dispenser to dispense a medication or substance to the patient.

14. A method as claimed in any of claims 10-13, wherein the method further comprises determining the desired phase shift based on the shift factor and a desired number of days over which the cancer treatment is to be administered to the patient.

15. A computer program product comprising a computer readable medium having computer readable code embodied therein, the computer readable code being configured such that, on execution by a suitable computer or processor, the computer or processor is caused to perform the method of any of claims 10-14.

Fig. 1

Fig. 2

17

Fig. 3

EP 3 586 919 A1

|  | Baseline Day 1 | Day 2 | Day 3 | Day 4 | Day 5 | Day 6 | Day 7 | Day 8 | Day 9 | Day 10 |
|---|---|---|---|---|---|---|---|---|---|---|
| Healthy cells | 03:00 | 03:00 | 05:00 | 07:00 | 09:00 | 11:00 | 13:00 | 15:00 | 15:00 | 15:00 |
| Cancer cells | 03:00 | 03:00 | 03:00 | 03:00 | 05:00 | 07:00 | 09:00 | 11:00 | 13:00 | 15:00 |
| Treatment time window duration | 0 hr | 0 hr | 2 hr | 4 hr | 4 hr | 4 hr | 4 hr | 4 hr | 2 hr | 0 hr |
| Treatment time window | - | - | 02:00-04:00 | 01:00-05:00 | 03:00-07:00 | 05:00-09:00 | 07:00-11:00 | 09:00-13:00 | 11:00-13:00 | - |

Fig. 4

EP 3 586 919 A1

Fig. 5

Europäisches
Patentamt

European
Patent Office

Office européen
des brevets

## EUROPEAN SEARCH REPORT

Application Number

EP 18 18 0559

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SILKE KIESSLING ET AL: "The tumor circadian clock: a new target for cancer therapy?", FUTURE ONCOLOGY, vol. 13, no. 29, 1 December 2017 (2017-12-01), pages 2607-2610, XP055534435, GB ISSN: 1479-6694, DOI: 10.2217/fon-2017-0456 * the whole document * | 1-15 | INV. A61N5/10 A61N5/06 |
| A | Loning Fu ET AL: "The Circadian Clock in Cancer Development and Therapy" In: "Progress in molecular biology and translational science", 1 January 2013 (2013-01-01), Elsevier, Netherlands, XP055534843, ISSN: 1877-1173 vol. 119, pages 221-282, DOI: 10.1016/B978-0-12-396971-2.00009-9, * the whole document * | 1-15 | |
| | | | TECHNICAL FIELDS SEARCHED (IPC) |
| A | S. CHAN ET AL: "Does the Time of Radiotherapy Affect Treatment Outcomes? A Review of the Literature", CLINICAL ONCOLOGY, vol. 29, no. 4, 1 April 2017 (2017-04-01), pages 231-238, XP055534070, AMSTERDAM, NL ISSN: 0936-6555, DOI: 10.1016/j.clon.2016.12.005 * the whole document * | 1-15 | A61N |

-/--

The present search report has been drawn up for all claims

| Place of search | Date of completion of the search | Examiner |
|---|---|---|
| Munich | 14 December 2018 | Beck, Ewa |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | STEPHANIE CHAN ET AL: "Effects of circadian rhythms and treatment times on the response of radiotherapy for painful bone metastases", ANNALS OF PALLIATIVE MEDICINE, vol. 6, no. 1, 1 January 2017 (2017-01-01), pages 14-25, XP055534073, ISSN: 2224-5820, DOI: 10.21037/apm.2016.09.07 * the whole document * | 1-15 | |
| A | WO 2013/132454 A1 (KONINKL PHILIPS NV [NL]) 12 September 2013 (2013-09-12) * the whole document * | 1-15 | |
| A | WO 95/05819 A1 (OREGON STATE [US]) 2 March 1995 (1995-03-02) * abstract * * page 10, line 23 - page 11, line 26 * | 1-15 | |

TECHNICAL FIELDS
SEARCHED      (IPC)

The present search report has been drawn up for all claims

| Place of search | Date of completion of the search | Examiner |
|---|---|---|
| Munich | 14 December 2018 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 586 919 A1**

## ANNEX TO THE EUROPEAN SEARCH REPORT
## ON EUROPEAN PATENT APPLICATION NO.

EP 18 18 0559

14-12-2018

| Patent document cited in search report | | | Publication date | Patent family member(s) | | | Publication date |
|---|---|---|---|---|---|---|---|
| WO 2013132454 | A1 | | 12-09-2013 | CN | 104160399 | A | 19-11-2014 |
| | | | | EP | 2823423 | A1 | 14-01-2015 |
| | | | | JP | 2015520427 | A | 16-07-2015 |
| | | | | US | 2015037769 | A1 | 05-02-2015 |
| | | | | WO | 2013132454 | A1 | 12-09-2013 |
| WO 9505819 | A1 | | 02-03-1995 | AU | 7603094 | A | 21-03-1995 |
| | | | | US | 5591768 | A | 07-01-1997 |
| | | | | US | 6069164 | A | 30-05-2000 |
| | | | | US | 6423738 | B1 | 23-07-2002 |
| | | | | US | 2003008912 | A1 | 09-01-2003 |
| | | | | US | 2006165786 | A1 | 27-07-2006 |
| | | | | WO | 9505819 | A1 | 02-03-1995 |

EPO FORM P0459

For more details about this annex : see Official Journal of the European Patent Office, No. 12/82